# EUROPEAN PATENT SPECIFICATION

(11) **EP 1 859 745 B1**
(45) Date of publication and mention of the grant of the patent: **17.11.2010**
(21) Application number: 07252169.3
(22) Date of filing: 25.05.2007
(51) Int. Cl.: A61B 17/068, A61B 17/072, A61B 17/128, A61F 5/00, A61B 17/00, A61B 17/30

(54) **Endoscopic gastric restriction device**
Vorrichtung zur endoskopischen Magenbegrenzung
Dispositif endoscopique de restriction gastrique

(30) Priority: 25.05.2006 US 420365
(43) Date of publication of application: 28.11.2007
(73) Proprietor: ETHICON ENDO-SURGERY, INC., Cincinnati, Ohio 45242 (US)
(72) Inventor: Ortiz, Mark S., Milford Ohio (US); Plescia, David N., Cincinnati Ohio (US); Ditter, Tom Allen, Fremong California (US); Eke, Soane H., Palo Alto California (US)
(74) Representative: Tunstall, Christopher Stephen

(56) References cited:
- WO-A-02/096327
- WO-A-2004/050971
- WO-A2-2005/079673

## Description

### FIELD OF THE INVENTION

The present invention relates broadly to devices for endoscopically performing gastric restriction surgery.

### BACKGROUND OF THE INVENTION

Morbid obesity is a serious medical condition. In fact, morbid obesity has become highly pervasive in the United States, as well as other countries, and the trend appears to be heading in a negative direction. Complications associated with morbid obesity include hypertension, diabetes, coronary artery disease, stroke, congestive heart failure, multiple orthopedic problems and pulmonary insufficiency with markedly decreased life expectancy. With this in mind, and as those skilled in the art will certainly appreciate, the monetary and physical costs associated with morbid obesity are substantial. In fact, it is estimated the costs relating to obesity are in excess of 100 billion dollars in the United States alone.

A variety of surgical procedures have been developed to treat obesity. The most common currently performed procedure is Roux-en-Y gastric bypass (RYGB). This procedure is highly complex and is commonly utilized to treat people exhibiting morbid obesity. However, and with this in mind, greater than 100,000 procedures are performed annually in the United States alone. Other forms of bariatric surgery include Fobi pouch, bilio-pancreatic diversion, and gastroplastic or "stomach stapling". In addition, implantable devices are known which limit the passage of food through the stomach and affect satiety.

RYGB involves movement of the jejunum to a high position using a Roux-en-Y loop. The stomach is completely divided into two unequal portions (a smaller upper portion and a larger lower gastric pouch) using an automatic stapling device. The upper pouch typically measures less than about or 20 cc, (1 ounce), while the larger lower pouch remains generally intact and continues to secrete stomach juices flowing through the intestinal track.

A segment of the small intestine is then brought from the lower abdomen and joined with the upper pouch to form an anastomosis created through a 12.5 mm or half-inch opening, also called the stoma. This segment of the small intestine is called the "Roux loop" and carries the food from the upper pouch to the remainder of the intestines, where the food is digested. The remaining lower pouch, and the attached segment of duodenum, are then reconnected to form another anastomotic connection to the Roux loop at a location approximately 50 to 150 cm from the stoma, typically using a stapling instrument. It is at this connection that the digestive juices from the bypass stomach, pancreas, and liver, enter the jejunum and ileum to aid in the digestion of food. Due to the small size of the upper pouch, patients are forced to eat at a slower rate and are satiated much more quickly. This results in a reduction in caloric intake.

The conventional RYGB procedure requires a great deal of operative time. Because of the degree of invasiveness, post-operative recovery time can be quite lengthy and painful. In view of the highly invasive nature of the current RYGB procedure, other less invasive procedures have been developed. The most common form of gastric reduction surgery is a gastric restriction, which involves the application of vertical staples along the stomach to create an appropriate pouch. This procedure is commonly performed laparoscopically and, as such, requires substantial preoperative, operative, and postoperative resources.

WO 02/096327 discloses an endoscopic gastric restriction device according to the preamble of claim 1.

With the foregoing in mind, procedures that allow for the performance of gastric reduction surgery in a time efficient and patient friendly manner are needed. Accordingly, the present invention provides devices for performing a gastric restriction.

### SUMMARY OF THE INVENTION

The present invention generally provides devices for endoscopically performing a gastric restriction. In one exemplary embodiment, an endoscopic gastric restriction device is provided having an elongate end effector with first and second troughs extending longitudinally therethrough and adapted to receive tissue therein, and a plurality of fasteners disposed within the end effector and configured to self-deploy to engage tissue disposed within the trough when the fasteners are released from the end effector. The end effector can have a variety of configurations, and in one embodiment it can be formed from a plurality of segments that are movably coupled to one another to allow the end effector to flex as it passes through a tortuous lumen. The device can also include a suture coupled to the plurality of fasteners and pre-woven such that the suture will extend through the plurality of fasteners when the fasteners are released from the end effector to engage tissue. Each fastener can also have a variety of configurations. For example, each fastener can include opposed legs that come together to form a ring-shaped member when the fasteners are released from the end effector.

In another embodiment, an endoscopic gastric restriction device is provided and includes a shaft having an end effector on a distal end thereof. The end effector can include a plurality of segments movably coupled to one another. The segments can be movably coupled to one another by, for example, at least one elongate flexible member extending through the plurality of segments. The end effector can also include at least one trough formed in the plurality of segments and adapted to receive tissue therein. In an exemplary embodiment, the end effector includes first and second troughs formed in and extending along opposed sides of the plurality of segments. The device can further include at least one fastener-retaining member extending through the plurality of segments and adapted to retain at least one tissue fastener and to deliver the at least one tissue fastener to tissue disposed within the at least one trough. In one embodiment, the device includes first and second fastener-retaining members extending through the end effector on opposed sides of the first trough, and third and fourth fastener-retaining members extending through the end effector on opposed sides of the second trough. The fastener-retaining member(s) can be slidably movable along an axis of the end effector between a first position, in which the fastener-retaining member(s) retains opposed legs of at least one fastener on opposed sides of a trough, and a second position, in which the fastener-retaining member(s) releases the opposed legs of the at least one fastener. The fastener-retaining member(s) can be coupled to at least one actuator adapted to move the fastener-retaining member(s) between the first and second positions. In certain exemplary embodiments, the actuator(s) can be adapted to retract in length when heat is applied thereto thereby slidably moving the fastener-retaining member(s) between the first and second positions.

The device can include other features, such as at least one tissue injuring element disposed within the at least one trough and adapted to injure tissue positioned therein, an optical image gathering unit disposed on a portion of the end effector for viewing a surgical site surrounding the end effector, and/or a plurality of suction ports formed within the at least one trough for suctioning tissue therein. Where the end effector includes a plurality of suction ports, the device can optionally include a first suction tube configured to apply suction through a plurality of suction ports formed in the first trough, and a second suction tube configured to apply suction through a plurality of suctions ports formed in the second trough, thereby allowing for differential suction. In other embodiments, the end effector can be movably coupled to the shaft. For example, a four-bar linkage mechanism can movably couple the end effector to the shaft.

The device can also include a plurality of fasteners retained within the end effector and extending across the trough for engaging tissue positioned in the trough. In an exemplary embodiment, the fasteners can be formed from a super elastic material and they can be configured to engage tissue when they are released by the at least one fastener-retaining member. At least one suture can extend through the end effector and it can be configured to couple to the plurality of fasteners upon deployment thereof into tissue.

The present disclosure also provides exemplary methods for creating a gastric restriction using the device of the invention. In one embodiment, the method can include introducing an elongate member through an esophagus to position an end effector on the elongate member within a stomach. The end effector can flex as it passes through the esophagus. The method can further include suctioning tissue into a trough formed in the end effector, delivering a plurality of fasteners to the tissue disposed within the trough, and cinching a suture coupled to the plurality of fasteners. The suture is preferably pre-woven through the fasteners before the fasteners are delivered. In an exemplary embodiment, a tip of the end effector can be positioned within the pylorus to anchor the end effector within the stomach. In another embodiment, the stomach can be insufflated after the fasteners are delivered to separate the fasteners from the end effector.

Various techniques can be used to deliver the fasteners. For example, the fasteners can be delivered by axially sliding at least one firing bar extending through the end effector to release the plurality of fasteners. In an exemplary embodiment, the fasteners are delivered by releasing a first leg of the plurality of fasteners to cause the first leg to penetrate into tissue disposed within the trough, and releasing a second leg of the plurality of fasteners to cause the second leg to penetrate into tissue to form a ring-shaped fastener with the first leg that engages the tissue. In other embodiments, the plurality of fasteners can be delivered simultaneously. The method can also include applying energy to at least one tissue injuring element formed within the trough to injure the tissue in the trough.

### BRIEF DESCRIPTION OF THE DRAWINGS

The invention will be more fully understood from the following detailed description taken in conjunction with the accompanying drawings, in which:

FIG. 1 is a perspective view of one exemplary embodiment of an endoscopic gastric restriction device having an elongate shaft with an end effector including a plurality of segments;

FIG. 2 is an exploded view of a portion of the end effector of FIG. 1, showing members for flexibly mating the segments;

FIG. 3 is a perspective view of a portion of the endoscopic gastric restriction device of FIG. 1, showing an articulating connection between the end effector and the elongate shaft of the device;

FIG. 4 is a perspective view of one of the segments of the end effector of the endoscopic gastric restriction device of FIG. 1;

FIG. 5 is a side view of one exemplary embodiment of a fastener for use with the device of FIG. 1;

FIG. 6 is a cross-sectional view of a segment of the end effector of the endoscopic gastric restriction device of FIG. 1, showing the fastener of FIG. 5 disposed therein;

FIG. 7 is a partially disassembled view of a segment of the end effector of the endoscopic gastric restriction device of FIG. 1, showing a firing mechanism for releasing a plurality of fasteners from the end effector;

FIG. 8 is a distal perspective view of the endoscopic gastric restriction device of FIG. 1, showing one embodiment of optics disposed on the device to facilitate positioning of the device;

FIG. 9A is a partially cut-away view of a stomach showing the device of FIG. 1 disposed therein;

FIG. 9B illustrates the stomach of FIG. 9A with the device removed and leaving a plurality of fasteners disposed in the stomach and coupled by suture; and

FIG. 9C illustrates the stomach of FIG. 9B with the suture cinched to attached opposed tissue walls of the stomach.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention generally provides endoscopic devices for attaching opposed walls of tissue. The devices can be used to deliver a plurality of fasteners to opposed tissues, and suture extending through the fasteners can be used to pull the tissues together. The suture can be pre-woven through the fasteners, thus allowing for a quick and simple deployment. While the various devices disclosed herein can be used in a variety of procedures, in certain exemplary embodiments the devices are used to pull together opposed walls of the stomach to form a stomach pouch within the stomach which reduces the rate of gastric emptying. A person skilled in the art will appreciate that the methods and devices can be configured for use in virtually any medical procedure in which it is desirable to attach two tissue surfaces to one another.

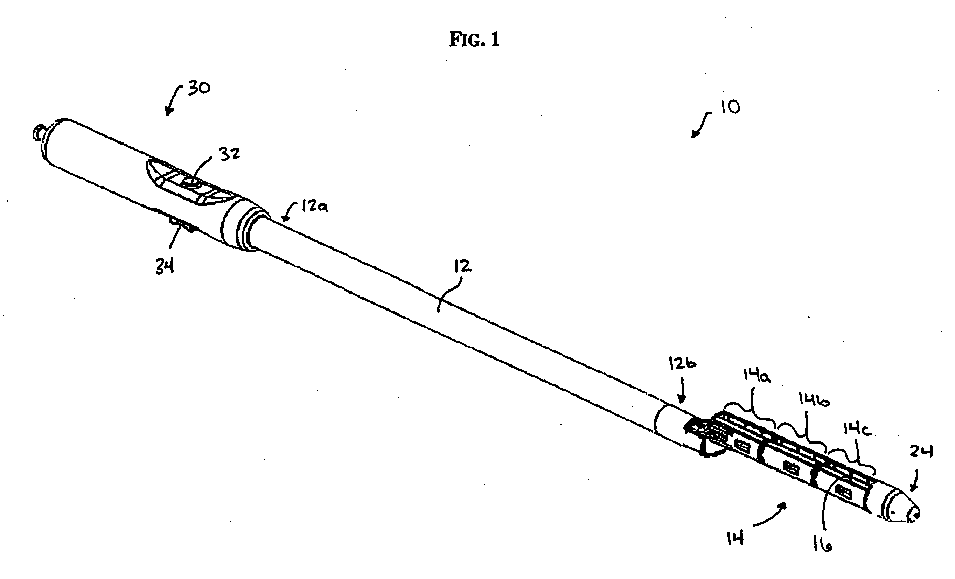

FIG. 1 illustrates one exemplary embodiment of a device 10 for attaching opposed tissue surfaces to one another. As shown, the device 10 generally includes an elongate shaft 12 having an end effector 14 located on a distal end 12b thereof. The end effector 14 includes opposed troughs (only one trough 16a is shown in FIG. 1) formed therein and configured to suction and pull opposed walls of tissue into the troughs. Each trough is also configured to deliver a plurality of fasteners to the tissue disposed therein. As a result, the end effector 14 can deliver multiple fasteners to opposed walls of tissue. The fasteners can be mated by a suture such that the suture can be cinched to pull the opposed walls of tissue together. Where the fasteners are delivered to the stomach, attachment of the opposed walls of the stomach will form a stomach pouch that restricts the passage of food. A person skilled in the art will appreciate that the methods and devices disclosed herein are not limited to use in performing a gastric restriction, and they may be applied in various soft tissue apposition procedures where tissue is drawn together either permanently or temporarily.

The elongate shaft 12 of the device 10 can have a variety of configurations, and the particular configuration can vary depending on the intended use of the device. For example, the device can be configured for use in laparoscopic applications, and thus the shaft can have a generally elongate rigid configuration that allows the shaft to be inserted through an access port or other port. Alternatively, the elongate shaft 12 can have a flexible configuration that allows the shaft 12 to be used endoscopically and thus inserted through a body lumen, such as the esophagus or colon. The shaft 12 can be formed from various materials and it can have various configurations that allow the shaft 12 to flex.

The shaft 12 can also include a handle 30 coupled to a proximal end 12a thereof. The handle 30 can facilitate grasping of the device 10, and it can also include various actuation mechanisms formed thereon to facilitate operation of the device 10. As shown in FIG. 1, the handle 30 includes a button 32 that is configured to be depressed to release a plurality of fasteners from the end effector 14, and a sliding lever 34 that is configured to move relative to the handle 30 to articulate the end effector 14 relative to the elongate shaft 12, as will be discussed in more detail below. The handle 30 can also include an opening formed in a proximal end thereof to allow various devices to be inserted through the handle 30 and the elongate shaft 12. A person skilled in the art will appreciate that other actuation mechanisms can be used depending on the configuration of the device 10.

The end effector 14, which is coupled to or formed on the distal end 12b of the shaft 12, can also have a variety of configurations, but in one exemplary embodiment the end effector 14 has a generally elongate cylindrical configuration to allow the end effector 14 to be inserted translumenally, i.e., through a body lumen. The end effector 14, or at least a portion thereof, can also be flexible to facilitate insertion through a tortuous body lumen. While various techniques can be used to provide a flexible end effector 14, in one exemplary embodiment, as shown in FIG. 1, the end effector 14 can include several segments 14a, 14b, 14c that are movably coupled to one another to allow the end effector 14 to flex and turn as the end effector 14 is inserted translumenally. While the particular quantity of segments can vary depending on the length of each segment and the intended use of the device 10, FIG. 1 illustrates three segments 14a, 14b, 14b that are movably coupled to one another. Each segment 14a, 14b, 14b has a length that is about 50 mm or less, however the length can vary depending on the application. The end effector 14 can also include a distal tip or end cap 24 formed thereon or coupled thereto to facilitate insertion through a body lumen. As shown in FIG. 1, the end cap 24 has a generally conical configuration and it is coupled to the distal-most end of the third segment 14c.

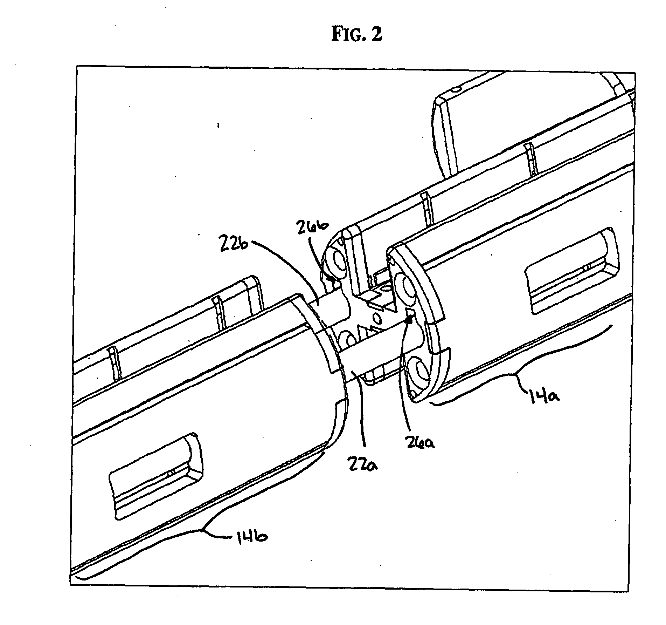

Various techniques can be used to movably mate the segments 14a, 14b, 14c to one another, but in one exemplary embodiment, shown in more detail in FIG. 2, the segments 14a, 14b, 14c can be connected by one or more flexible elongate members. In particular, FIG. 2 illustrates two elongate flexible springs 22a, 22b extending through longitudinal bores 26a, 26b formed through each segment (FIG. 2 only illustrates segments 14a and 14b). The springs 22a, 22b mate to the first segment 14a at the proximal-most end thereof and to the last or third segment 14c at the distal most end thereof. The springs 22a, 22b can alternatively mate to the end cap 24 shown in FIG. 1, rather than the last or third segment 14c. In use, the segments 14a, 14b, 14c are free to slide and bend relative to the springs 22a, 22b, thus allowing the segments 14a, 14b, 14c to move relative to one another. While springs are shown, flexible shafts, cables, or other flexible members can be used. Moreover, a person skilled in the art will appreciate that a variety of other techniques can be used to allow movement between the segments 14a, 14b, 14b.

Referring back to FIG. 1, the end effector 14 is shown having a generally linear configuration when the segments 14a, 14b, 14c are in a resting state. In other embodiments, the segments 14a, 14b, 14c can be configured to have a predetermined non-linear configuration, such as an S-shaped or C-shaped configuration, that the end effector 14 will assume in the resting state. This can be achieved by forming one or more of the segments 14a, 14b, 14c to have a curved configuration, rather than a linear configuration. Thus, when the end effector 14 is inserted through a tortuous lumen, the segments 14a, 14b, 14c can move relative to one another to allow the end effector 14 to pass through the lumen. Once the end effector 14 is positioned, for example within the patient's stomach, the end effector 14 can assume its predetermined non-linear configuration. This will allow the fasteners to be applied in a desired pattern or configuration.

In addition to the segments 14a, 14b, 14c of the end effector 14 being movable relative to one another, the end effector 14 can also be movable relative to the elongate shaft 12. For example, in one embodiment the end effector 14 can be configured to articulate relative to the distal end 12b of the elongate shaft 12 to allow the end effector 14 to be positioned at an angle relative to the axis of the elongate shaft 12. In another embodiment, the end effector 14 can be configured to move from a linear configuration, in which the end effector 14 is aligned with and extends along the longitudinal axis of the shaft 12, to an offset position in which the end effector 14 is positioned offset from the axis of the shaft 12, as shown in FIG. 1. In the offset position, the longitudinal axis of the end effector 14 can remain substantially parallel to the longitudinal axis of the elongate shaft 12. This is particularly advantageous where the elongate shaft 12 is used to introduce other devices. For example, the elongate shaft 12 can include an inner lumen extending therethrough for receiving an endoscopic device, such as an endoscope. Positioning the end effector 14 offset from the distal end 12b of the shaft 12 will prevent the end effector 14 from interfering with use of the endoscope, or other device.

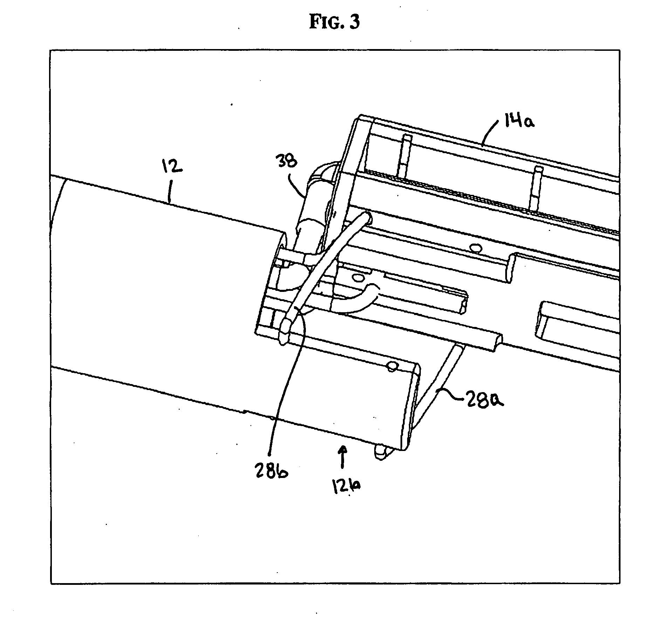

While virtually any technique known in the art for pivoting or articulating an end effector 14 relative to an elongate shaft 12 can be used with the present invention, in one exemplary embodiment the end effector 14 can be mated to the elongate shaft 12 using a four-bar linkage mechanism. This is illustrated in more detail in FIG. 3, which shows first and second linkages 28a, 28b extending between the distal end 12b of the elongate shaft 12 and the proximal end of the first segment 14a. The linkages 28a, 28b are mated to the shaft 12 and segment 14a at a distance apart from one another such that proximally-directed tension applied to an actuator (not shown), such as a cable or other mechanism, extending through the elongate shaft 12 and coupled to the end effector 14 will pull the end effector 14 proximally thereby moving the end effector 14 to one side of the elongate shaft 12. The linkages 28a, 28b can also be formed from a shape memory material or other spring material such that release of the actuator will allow the linkages 28a, 28b to apply a force to the end effector 14 to move the end effector 14 to an initial linear configuration. Alternatively, the actuator can be configured to move the end effector 14 between the initial and offset positions.

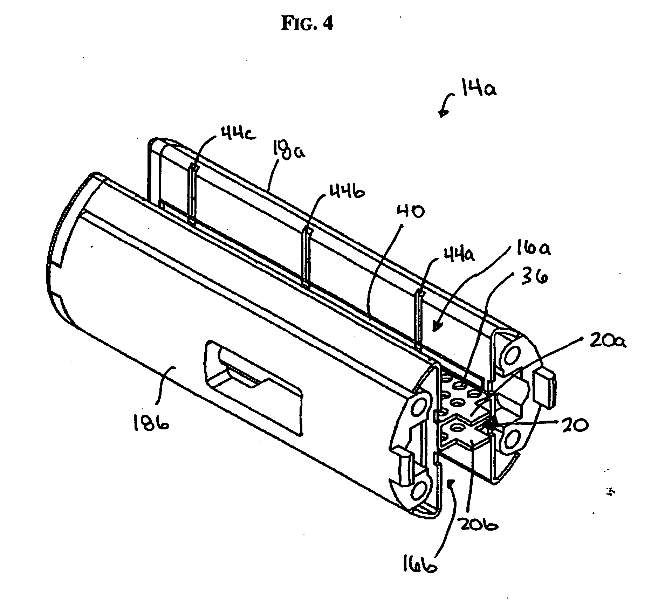

As previously indicated, the end effector 14 is configured to receive tissue in opposed troughs formed therein, and to deliver one or more fasteners to the tissue to allow two surfaces of tissue to be mated. Each segment 14a, 14b, 14c can have various configurations to allow tissue to be received therein and to deliver one or more fasteners to the tissue. However, FIG. 4 illustrates one exemplary embodiment of one of the segments, e.g., segment 14a. While only segment 14a is discussed, one skilled in the art will appreciate that the various other segments, e.g., segments 14b and 14c, can each have the same configuration.

As shown in FIG. 4, the segment 14a has a generally elongate configuration with opposed semi-cylindrical shaped sidewalls 18a, 18b that are connected by a transverse central portion 20 such that the sidewalls and central portion 20 define opposed superior and inferior troughs 16a, 16b in the segment 14a. The troughs 16a, 16b extend longitudinally along the length of the segment 14a (i.e., between proximal and distal ends of the segment) such that, when the segments 14a, 14b, 14c are mated, the end effector 14 includes a first or superior elongate trough formed in the first side thereof and a second or inferior elongate trough formed in the second, opposed side thereof. This will allow opposed walls of tissue to be engaged by the end effector 14, as will be discussed in more detail below. The particular width, depth, and shape of each trough 16a, 16b can vary, but each trough 16a, 16b preferably has a size that allows tissue to be received therein and to be engaged by a fastener contained within the end effector 14, as will be discussed below. In the illustrated embodiment, each trough 16a, 16b has a generally square or rectangular cross-sectional shape.

In order to position tissue within each trough 16a, 16b, the troughs 16a, 16b can include one or more suction ports formed therein and configured to suction tissue into the troughs 16a, 16b. As shown in FIG. 4, the central portion 20 includes superior and inferior members 20a, 20b each having a plurality of suction ports 36 formed therein. The superior and inferior members 20a, 20b are spaced a distance apart from one another to define a cavity that can receive a suction force therebetween to suction tissue into the troughs 16a, 16b, as will be discussed below. The superior and inferior members 20a, 20b can also be separated from one another by a divider to allow suction to be selectively applied to only one of the superior and inferior troughs 16a, 16b. In such a case, separate suction lines can extend through the end effector 14 for selectively suctioning tissue into the superior and/or inferior troughs 16a, 16b. This may be particularly useful where the tissues surfaces being attached to one another are spaced a distance apart from one another. The end effector 14 could be positioned adjacent to a first tissue surface, and suction can be applied to the first trough 16a to pull the first tissue surface into the trough 16a. The end effector 14 can then be moved, with the first tissue surface retained therein, toward a second tissue surface, and suction can be applied to the second trough 16b to pull the second tissue surface into the trough 16b. While not shown, the troughs 16a, 16b can include suction ports formed at various other locations, such as on the inner surfaces of the opposed sidewalls 18a, 18b of the segment 14a.

In order to generate suction within the end effector 14, the device 10 can include a suction line 38, shown in FIG. 3, extending through the elongate shaft 12 and coupled to the proximal end of the first segment 14a. The suction line 38 can terminate at the proximal end of the end effector 14, and it can be configured to generate a suction force through the entire length of the end effector 14. Alternatively, the suction line 38 can extend between the superior and inferior members of each segment 14a, 14b, 14c such that the suction line 38 extends through the length of the end effector 14. The suction line 38 can have multiple openings formed therein for generating a suction force along the length of the end effector 14. A person skilled in the art will appreciate that a variety of other techniques can be used to generate suction to pull tissue into the troughs in the end effector 14.

Continuing to refer to FIG. 4, the segment 14a can also optionally include a tissue injuring element disposed within the troughs 16a, 16b and configured to intentionally cause damage to tissue to promote healing after the tissue is apposed. While various tissue injuring techniques can be used, in one exemplary embodiment the tissue injuring elements can be in the form of electrical elements, such as electrodes for delivering RF, monopolar, bipolar, or other energy to the tissue, or mechanical elements, such as scrapers or little blades located on the sidewalls or back walls of the troughs 16a, 16b that move to cut the tissue. In an exemplary embodiment, the tissue injuring elements are in the form of two bipolar or monopolar strips that are disposed on the inner surfaces of the opposed sidewalls 18a, 18b of each trough 16a, 16b. FIG. 4 illustrates a tissue injuring strip 40 disposed on the inner surface extending along the length of the first sidewall 18a of the segment 14a. Alternatively, a portion of each sidewall 18a, 18b can be formed from a conductive material for receiving energy. The location of the tissue injuring elements on the sidewalls 18a, 18b allows the applied energy to travel across or between the walls of the trough 16a, 16b. The tissue injuring elements can, however, be positioned at various other locations, such as on the central member 20 or in particular zones. In use, energy can be delivered to the tissue injuring elements using, for example, one or more leads extending through the elongate shaft 12. A proximal end of each lead can be coupled to an internal or external energy source.

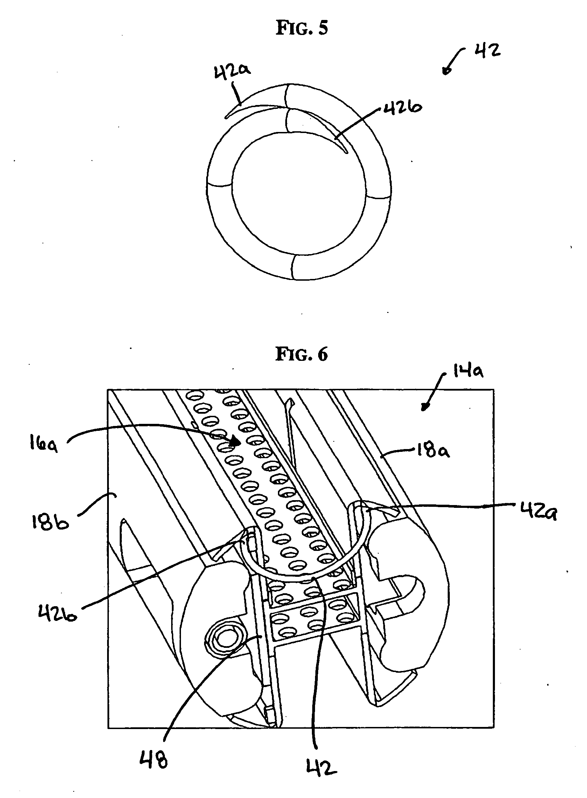

As previously indicated, once tissue is suctioned into the troughs 16a, 16b, the device 10 is configured to deliver one or more fasteners to the tissue. The particular technique used to deliver the fasteners can vary depending on the configuration of the fasteners, but in one exemplary embodiment the fasteners are configured to be self-deployed. FIG. 5 illustrates one exemplary self-deploying fastener 42 shown in a closed or deployed configuration. As shown, the fastener 42 includes opposed terminal ends 42a, 42b that are configured to penetrate through tissue and that form a circular or ring-shaped member in the closed position. Each end 42a, 42b can have a pointed or sharp tip to help penetrate through the tissue. In order to allow the fastener 42 to be self-deployed, the fastener 42 can be biased to the closed position, for example, by forming the fastener 42 from a super elastic or shape memory material. In use, the opposed ends 42a, 42b can be pulled apart to move the fastener 42 to an open position, and upon release of the opposed ends 42a, 42b the ends 42a, 42b will automatically move toward one another to penetrate through and tissue disposed within the trough.

Various techniques can be used to retain one or more fasteners 42 in the end effector 14, but in an exemplary embodiment each segment includes at least one and preferably multiple fastener-retaining cut-outs formed therein. FIG. 4, for example, illustrates three fastener-retaining cut-outs 44a, 44b, 44c formed therein such that the first segment 14a will deliver three fasteners to tissue disposed within the superior trough 16a. While not shown, the opposed inferior trough 16b likewise includes three fastener-retaining cut-outs formed therein for delivering three fasteners to tissue disposed within the opposed trough 16b. The cut-outs 44a, 44b, 44c are sized to allow the ends 42a, 42b of a fastener 42 to be retained therein, and to be selectively released to be deployed into tissue. FIG. 6 illustrates a fastener 42 having opposed ends 42a, 42b pulled apart from one another and positioned within one of the cut-outs, e.g., cut-out 44a, formed in the first segment 14a. The mid-portion of the fastener 42 is shown extending across the trough 16a such that tissue will be positioned between the ends 42a, 42b of the fastener 42 when tissue is suctioned into the trough 16a.

In order to retain the opposed ends 42a, 42b of the fastener 42 in the cut-out 44a, the device 10 can include one or more firing bars extending through each segment 14a, 14b, 14c and adapted retain and selectively release the fasteners from the end effector 14. While the firing bar(s) can have a variety of configurations, in an exemplary embodiment each segment 14a, 14b, 14c includes a first firing bar that is positioned within the first sidewall thereof for retaining a first end of each fastener disposed within the superior and inferior troughs, and a second firing bar that is positioned within the second sidewall thereof for retaining a second end of each fastener disposed within the superior and inferior troughs. This is illustrated, for example, in FIG. 6 which shows a firing bar 48 disposed within the second sidewall 18b of the first segment 14a. The firing bar 48 will retain the second end 42b of each fastener 42 disposed within the superior trough 16a, and the second end 42b of each fastener 42 disposed within the inferior trough 16b. Another firing bar (not shown) is disposed within the first sidewall 18a and it retains the first end 42a of each fastener 42 disposed within the superior trough 16a, and the first end 42a of each fastener 42 disposed within the inferior trough 16b.

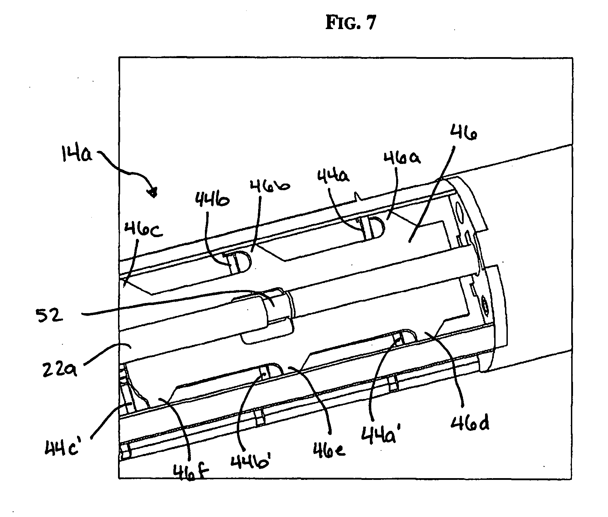

In order to retain the ends 42a, 42b of each fastener 42 within the cut-outs 44a, 44b, 44c, each firing bar can include a plurality of fastener-retaining legs formed therein. FIG. 7 illustrates a firing bar 46 that is disposed within the first sidewall 18a of the first segment 14a, and that includes six legs 46a, 46b, 46c, 46d, 46e, 46f formed therein. The firing bar 46 has a generally elongate planar configuration, and three legs 46a, 46b, 46c extend from a first side thereof toward the cut-outs (only two of the three cut-outs are shown 44a, 44b) formed in the superior trough 16a, and the other three legs 46d, 46e, 46f extend from an opposed side thereof toward the cut-outs 44a', 44b', 44c' formed in the inferior trough 16b. Each leg 46a-f includes a hook-shaped terminal end that is configured to engage one of the terminal ends 42a, 42b of a fastener 42 to hold the end in the cut-out. The hook-shaped terminal end of each leg 46a-46f also extends in the same direction, such that movement of the firing bar 46 relative to the segment 14a will simultaneously release all of the terminal ends of the fasteners engaged by the firing bar 46. Since the segment 14a includes two firing bars 46, 48, the firing bars 46a, 48 can be actuated separately to release the one of the ends, e.g., the first end 42a, of each fastener 42 prior to releasing the other end, e.g., the second end 42b, of each fastener 42. This is particularly advantageous as it can facilitate tissue penetration. In particular, where only one of the ends of the fastener is released at a time, the tissue in the trough will be sandwiched between the leg of the fastener and the opposite wall of the trough, thereby enhancing piercing.

As indicated above, the ends of the fasteners can be released by moving the firing bars 46, 48 within the segment 14a. In an exemplary embodiment, the firing bars 46, 48 are adapted to slide between proximal and distal positions along the axis of the segment 14a to move between an engaged position, in which the fasteners are retained within the end effector 14, and a disengaged position, in which the ends of the fasteners are released and are free to close to penetrate tissue disposed within the troughs 16a, 16b. Various techniques can be used to slide the firing bars 46, 48. For example, the device 10 can elongate one or more actuator rods extending therethrough and coupled to the firing bars 46, 48 for moving the firing bars between the proximal and distal positions. In another embodiment, energy (e.g., heat) can be delivered to shape memory alloy (SMA) wires that are coupled to the firing bars to contract the wires and thereby pushing the firing bars from a proximal position to a distal position to release the fasteners. For example, first and second wires can extend through the springs 22a, 22b or other flexible members that connect the segments 14a, 14b, 14c. The first wire can move the first firing bar in each segment 14a, 14b, 14c, and the second wire can move the second firing bar in each segment 14a, 14b, 14c. FIG. 7 illustrates the first spring 22a extending through the first segment 14a, and a connector 52 positioned at a mid-portion thereof and fixedly mated to the wire (not shown) extending through the spring 22a. The tab 52 extends into an opening formed in the firing bar 46 such that movement of the tab 52 in between proximal and distal positions will move the firing bar 46. In order to move the firing bars, energy can be selectively delivered to each wire via a lead extending through the elongate shaft 12 and coupled to the wire to cause the wire to contract. As previously explained, the handle 30 of the device 10 can include, for example, a button 32 that is configured to be depressed to activate an energy source disposed within or coupled to the handle and thereby deliver energy to the wires to move the firing bars. While not shown, the handle 30 can optionally include two separate buttons for delivering energy to two separate wires for individually moving the first firing bar in each segment and the second firing bar in each segment. This will allow the first end of each fastener in the superior and inferior troughs in the end effector to be simultaneously released, and to be released prior to simultaneously releasing the second end of each fastener in the superior and inferior troughs. A person skilled in the art will appreciate that a variety of other techniques can be used to move the firing bars.

While not shown, the device 10 can also include one or more sutures extending therethrough and positioned such that they will extend through each fastener when the fasteners are deployed into the tissue disposed within each trough. In an exemplary embodiment, a single suture extends through the elongate shaft 12 of the device, through the first trough between the ends of each fastener, through the second trough between the ends of each fastener, and back up through the elongate shaft 12. As a result, when all of the fasteners are released, the suture will extend through each of the fasteners, and the free ends of the suture will remain outside of the patient's body. This will allow the device to be removed, and the suture to be tensioned to mate the opposed tissue surfaces.

The device 10 can also include a variety of other features known in the art. For example, various tissue glues may also be employed to facilitate mating of the opposed tissue walls, and to seal the tissue together. By way of non-limiting example, a fibrin-based tissue glue can be disposed on the superior and inferior members of the central portion of each segment so as to contact the tissue being suctioned into the superior and inferior troughs. This glue reduces loading on the fasteners apposing tissue, increasing the effective holding duration.

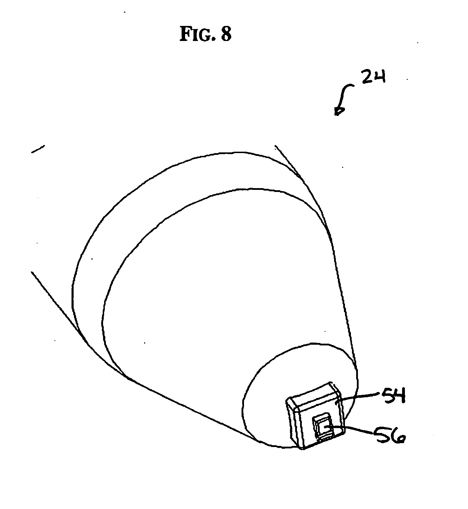

In another embodiment, the device 10 can include an optical image gathering unit disposed thereon and configured to acquire images during endoscopic procedures. While the location of the unit can vary, in one embodiment the optical image gathering unit can be disposed on the end cap 24, as shown in FIG. 8. In particular, FIG. 8 illustrates a housing 54 that protrudes from an outer surface of the end cap 24, and that contains the optical image gathering unit therein. A viewing window 56 is formed on a distal-facing surface of the housing 54 to allow the unit to acquire images of the end effector 14 and surrounding surgical site. The images from the optical image gathering unit can be transferred to an external image display screen, or alternatively the device 10 can include image display screen disposed on or coupled to a proximal portion of the device.

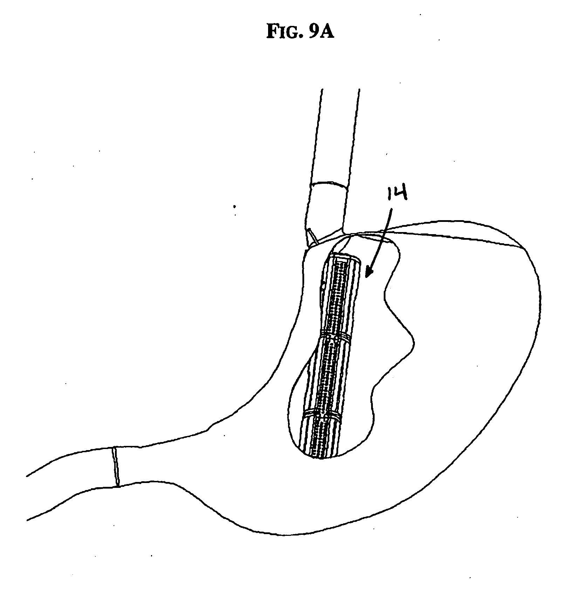

FIGS. 9A-9C illustrate one exemplary method for attaching opposed tissue surfaces to one another using, and in particular for creating a gastric restriction. The method is described in connection with the device 10 of FIG. 1, however various other devices can be used. As shown in FIG. 9A, the device 10 is introduced translumenally through the esophagus to position the end effector 14 within the stomach. The device 10 can be delivered along a guide wire pre-positioned within the stomach, and/or it can be delivered with the aid of an endoscope extending through the elongate shaft 12 of the device. For example, a steering mechanism on the endoscope can be used to steer the device through the body lumen. Once the end effector 14 is positioned within the stomach, the distal end of the device 10 can optionally be anchored within the pylorus valve by positioning the end cap 24 against the lesser curve of the stomach. This will help maintain the device 10 in a proper position for performing a gastric restriction. The end effector 14 can also be articulated relative to the elongate shaft 12 to position the end effector 14 offset from the elongate shaft 12. This can be achieved by sliding the lever 34 on the handle 30 to pull the actuator cable attached to the end effector 14 and thereby pivot the end effector 14.

Once the device 10 is in the desired position, suction can be applied to one or both troughs to pull tissue into the troughs. Suction applied to one of the troughs, e.g., the superior trough, will pull tissue on the anterior side of the stomach into the trough, and suction applied to the other trough, e.g., the inferior trough, will pull tissue on the posterior side of the stomach into the trough. Suction is preferably maintained at least until the fasteners are delivered.

Once both tissue surfaces are pulled into the troughs, the tissue can optionally be injured, and the fasteners can be delivered either before or after tissue injury. Tissue injury can be achieved by actuating the button or knob (not shown) on the handle that activates an energy source for delivering energy to the tissue injuring elements, and the fasteners can be delivered by actuating one or more buttons, e.g., button 32, on the handle 30. As previously explained, in an exemplary embodiment a first button is actuated to move the firing bars located on a first side of each of the superior and inferior troughs. When energy is delivered to the first wire, the wire will move the firing bars to release the first end of each fastener contained within the end effector. The released ends will penetrate through tissue disposed within the troughs, and they will likely extend across the trough to overlap with the second end and thereby form a ring-shaped fastener that engages the tissue disposed within the trough. A second button can then be actuated to move the firing bars located on a second side of each of the superior and inferior troughs. When energy is delivered to the second wire, the wire will move the firing bars to release the second end of each fastener contained with the end effectors, thereby releasing the fasteners from the troughs. Suction can be terminated to release the tissue with the fasteners disposed therein.

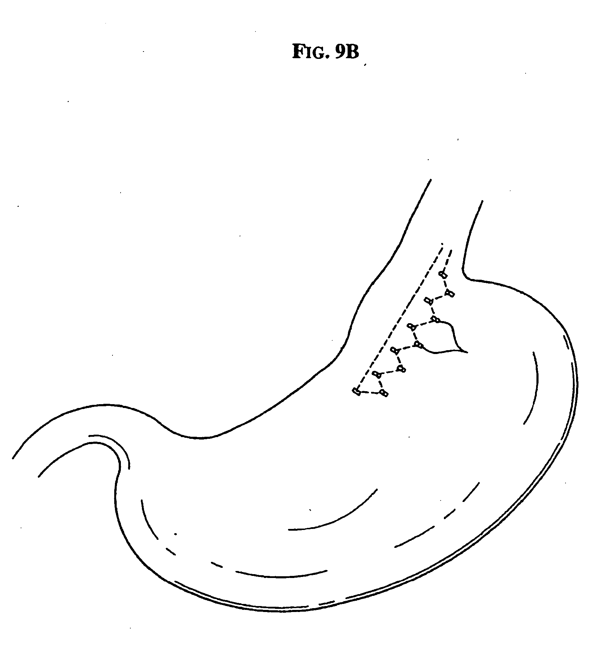

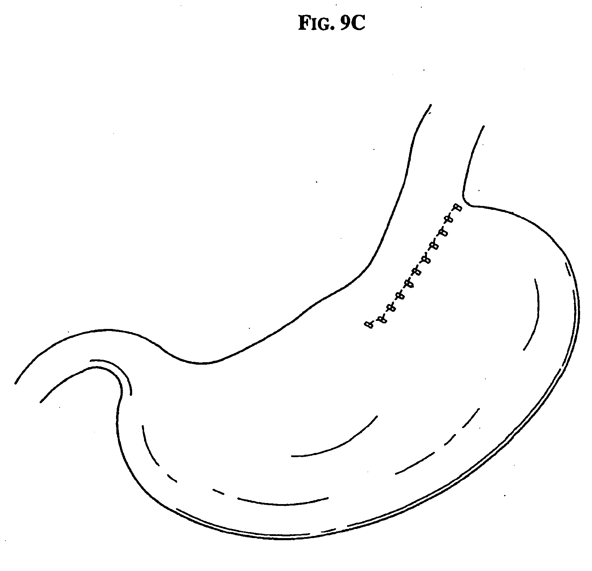

Once the fasteners are deployed into the anterior and posterior walls of the stomach, suction can be removed and replaced with insufflation. This pushes the stomach walls apart which can help to pull the fasteners out of their respective cut-outs within the end effector. The end effector can thereafter be extracted from the stomach cavity. What remains is a series of fasteners secured along the anterior and posterior walls of the stomach, and a pre-threaded suture extending through each of the fasteners. As shown in FIG. 9B, the fasteners can be delivered to form a stitch pattern, such as a mattress stitch, that extends from the proximal end to the distal end of the stomach. Other stitch patterns can be used, including a linear stitch pattern. In order to bring the anterior and posterior walls of the stomach together, tension can be applied to the free ends of the suture to cinch the suture, as shown in FIG. 9C. The free ends can be knotted or otherwise mated to one another to retain the suture in the cinched configuration. For example, a knotting element mounted on or positioned through the endoscope can be used to knot and cut the free ends. The resulting structure of the stomach is that of a small tubular pouch. The pouch may extend the full length between the esophagus and the pylorus or it may extend only a portion of the distance from the esophagus to the pylorus. Regardless of the length of the stitch, the gastric remnant allows gastric acid to pass into the food stream, while the small stomach pouch creates a reduced passageway that restricts the passage of food therethrough.

The various devices disclosed herein, including portions thereof, can be designed to be disposed after a single use, or can be designed to be used multiple times. In either case, however, the devices can be reconditioned for reuse after at least one use. Reconditioning can include any combination of the steps of disassembly of the device, followed by cleaning or replacement of particular pieces, and subsequent reassembly. By way of example, the device of FIG. 1 can be reconditioned after the device has been used in a medical procedure. The device can be disassembled, and any number of the particular pieces (e.g., the fasteners, suture, end effector, and tissue-injury elements) can be selectively replaced or removed in any combination. For example, the fasteners and sutures can be replaced by adding a new fastener cartridge to the end effector or by replacing the end effector. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

One skilled in the art will appreciate further features and advantages of the invention based on the above-described embodiments. Accordingly, the invention is not to be limited by what has been particularly shown and described, except as indicated by the appended claims.

## Claims

1. An endoscopic gastric restriction device, comprising:
an elongate end effector having first and second troughs extending longitudinally therethrough and adapted to receive tissue therein; **characterised in that** the device further comprises
a first plurality of fasteners disposed within the end effector and configured to self-deploy to engage tissue disposed within the first trough when released from the end effector;
a second plurality of fasteners disposed within the end effector and configured to self-deploy to engage tissue disposed within the second trough when released from the end effector and
a suture coupled to the fasteners and pre-woven such that the suture will extend through the first and second pluralities of fasteners when the fasteners are released from the end effector to engage tissue.

2. The device of claim 1, wherein the end effector is formed from a plurality of segments that are movably coupled to one another to allow the end effector to flex as it passes through a tortuous lumen.

3. The device of claim 1, wherein the first and second troughs include a plurality of suctions ports formed therein for suctioning tissue into the first and second troughs.

4. The device of claim 1, wherein the fasteners include opposed legs that come together to form a ring-shaped member when the fasteners are released from the end effector.

5. The device of claim 1, further comprising at least one tissue injuring element disposed within one of the troughs and adapted to injure tissue positioned therein.

6. A method for processing the device of claim 1 for surgery, comprising:
a) obtaining the device of claim 1;
b) sterilizing the device; and
c) storing the device in a sterile container.

7. A method of reconditioning the device of claim 1, comprising:
removing and replacing at least a portion of the device to prepare the device for re-use.

## Patentansprüche

1. Vorrichtung zur endoskopischen Magenbegrenzung, umfassend:
einen langgestreckten End-Manipulator mit ersten und zweiten Wannen, welche sich der Länge nach durch diesen erstrecken und adaptiert sind, um darin Gewebe aufzunehmen; **dadurch gekennzeichnet, dass** die Vorrichtung ferner umfasst
eine erste Vielzahl von Befestigungsmitteln, welche innerhalb des End-Manipulators angeordnet sind, und konfiguriert sind, um innerhalb der ersten Wanne angeordnetes Gewebe selbsttätig zu greifen, wenn sie vom End-Manipulator freigesetzt werden;
eine zweite Vielzahl von Befestigungsmitteln, welche innerhalb des End-Manipulators angeordnet sind, und konfiguriert sind, um innerhalb der zweiten Wanne angeordnetes Gewebe selbsttätig zu greifen, wenn sie vom End-Manipulator freigesetzt werden, und
ein Nahtmaterial, welches an die Befestigungsmittel gekoppelt und vorverwoben ist, so daß sich das Nahtmaterial durch die erste und die zweite Mehrzahl von Befestigungsmitteln erstrecken wird, wenn die Befestigungsmittel vom End-Manipulator freigesetzt werden, um das Gewebe zu greifen.

2. Vorrichtung nach Anspruch 1, wobei der End-Manipulator von einer Vielzahl von Segmenten gebildet wird, welche beweglich aneinander gekoppelt sind, um es dem End-Manipulator zu ermöglichen, sich zu verbiegen, wenn er durch ein gewundenes Lumen durchtritt.

3. Vorrichtung nach Anspruch 1, wobei die ersten und zweiten Wannen eine Vielzahl von darin ausgebildeten Saugöffnungen umfassen, um das Gewebe in die ersten und zweiten Wannen zu saugen.

4. Vorrichtung nach Anspruch 1, wobei die Befestigungsmittel gegen-tiberliegende Beine umfassen, welche zusammentreffen, um einen ringförmigen Teil auszubilden, wenn die Befestigungsmittel vom End-Manipulator freigesetzt werden.

5. Vorrichtung nach Anspruch 1, welche ferner wenigstens ein gewebeverletzendes Element umfasst, welches innerhalb einer der Wannen angeordnet ist, und adaptiert ist, um darin positioniertes Gewebe zu verletzen.

6. Verfahren zur Handhabung der Vorrichtung nach Anspruch 1 in der Chirurgie, umfassend:
a) Erhalten der Vorrichtung nach Anspruch 1;
b) Sterilisieren der Vorrichtung; und
c) Lagern der Vorrichtung in einem sterilen Behälter.

7. Verfahren zur Rekonditionierung der Vorrichtung nach Anspruch 1, umfassend:
Entfernen und Ersetzen von wenigstens einem Teil der Vorrichtung, um die Vorrichtung für die Wiederverwendung vorzubereiten.

## Revendications

1. Dispositif endoscopique de restriction gastrique, comprenant :
un organe terminal effecteur allongé présentant des première et seconde auges s'étendant longitudinalement à travers ledit organe et conçues pour y recevoir un tissu ; **caractérisé en ce que** le dispositif comprend en outre
une première pluralité d'éléments de fixation disposés à l'intérieur de l'organe terminal effecteur et configurés pour se déployer automatiquement pour venir en contact avec le tissu disposé à l'intérieur de la première auge lorsque ledit tissu est libéré de l'organe terminal effecteur ;
une seconde pluralité d'éléments de fixation disposés à l'intérieur de l'organe terminal effecteur et configurés pour se déployer automatiquement pour venir en contact avec le tissu disposé à l'intérieur de la seconde auge lorsque ledit tissu est libéré de l'organe terminal effecteur ; et
une suture couplée aux éléments de fixation et pré-tissée de telle sorte que la suture s'étend à travers les première et seconde pluralités d'éléments de fixation lorsque les éléments de fixation sont libérés de l'organe terminal effecteur pour venir en contact avec le tissu.

2. Dispositif selon la revendication 1, dans lequel l'organe terminal effecteur est composé d'une pluralité de segments couplés de manière amovible les uns aux autres pour permettre à l'organe terminal effecteur de fléchir à mesure qu'il passe à travers une lumière tortueuse.

3. Dispositif selon la revendication 1, dans lequel les première et seconde auges comportent une pluralité d'orifices d'aspiration formés dedans pour aspirer le tissu à l'intérieur des première et seconde auges.

4. Dispositif selon la revendication 1, dans lequel les éléments de fixation comportent des jambes opposées se réunissant pour former un élément annulaire lorsque les éléments de fixation sont libérés de l'organe terminal effecteur.

5. Dispositif selon la revendication 1, comprenant en outre au moins un élément de lésion tissulaire disposé à l'intérieur d'une des auges et conçu pour léser le tissu qui y est positionné.

6. Procédé de traitement du dispositif de la revendication 1 pour la chirurgie, comprenant les étapes consistant à :
a) obtenir le dispositif de la revendication 1 ;
b) stériliser le dispositif ; et
c) stocker le dispositif dans un contenant stérile.

7. Procédé de reconditionnement du dispositif de la revendication 1, comprenant l'étape consistant à :
déplacer et replacer au moins une partie du dispositif pour préparer le dispositif à sa réutilisation
